# EUROPEAN PATENT SPECIFICATION

(11) **EP 1 129 686 B1**
(45) Date of publication and mention of the grant of the patent: **06.07.2005**
(21) Application number: 01101963.5
(22) Date of filing: 29.01.2001
(51) Int. Cl.: A61K 7/02, A61K 7/06

(54) **Cleansing agents**
Reinigungsmittel
Produits de nettoyage

(30) Priority: 31.01.2000 JP 2000021556
(43) Date of publication of application: 05.09.2001
(73) Proprietor: SHISEIDO COMPANY LIMITED, Tokyo 104-8010 (JP)
(72) Inventor: Miyahara, Reiji, Shiseido Research Center, Yokohama-shi, Kanagawa 224-8558 (JP); Ohmori, Takashi, Shiseido Research Center, Yokohama-shi, Kanagawa 224-8558 (JP); Namba, Tomiyuki, Shiseido Research Center, Yokohama-shi, Kanagawa 224-8558 (JP); Kakoki, Hiroyuki, Shiseido Research Center, Yokohama-shi, Kanagawa 224-8558 (JP)
(74) Representative: TER MEER STEINMEISTER & PARTNER GbR

(56) References cited:
- EP-A- 0 813 860
- US-A- 5 437 860
- PATENT ABSTRACTS OF JAPAN vol. 017, no. 492 (C-1107), 7 September 1993 (1993-09-07) & JP 05 125396 A (NIPPON OIL & FATS CO LTD), 21 May 1993 (1993-05-21) & DATABASE WPI Derwent Publications Ltd., London, GB; AN 1993-200729
- PATENT ABSTRACTS OF JAPAN vol. 004, no. 042 (C-005), 3 April 1980 (1980-04-03) & JP 55 017310 A (SHIONO KORYO KK), 6 February 1980 (1980-02-06)

## Description

### Field of the Invention

The present invention relates to cleansing agents. More particularly, it relates to highly effective cleansing agents for removing makeup with excellent lathering, and for washing hairs providing smoothness of hair and refreshing after-shampoo feelings.

### Background of Invention

For the purpose of removing makeup cosmetics attached on skin, cleansing foams and facial cleansing gels prepared by surfactants as main ingredients, and cleansing oils comprising oil contents have been conventionally used.

However, cleansing foams and facial cleansing gels remove coats with silicone resins of makeup cosmetics by detergency of surfactants, and have merits of cleaning off neatly in addition to their excellent lathering, whereas their effects on makeup removing are insufficient.

Besides, although cleansing oils dissolve the coats of makeup cosmetics and are excellent for cleaning effects, they have had a problem where refreshing after-wash feeling is not given. As a concept, the aim is assumed to be achieved by combining oils with formulations in which surfactants are basis since silicone resins contained in makeup cosmetics are soluble in oils. However, general oils are not soluble in water, and surfactants are used to solubilize and emulsify these oils, thus this idea results in cleansing agents with poor lathering and refreshing feeling.

As a result of extensive studies on the above issues, the inventors of the present invention have found that the cleansing agents wherein surfactants are combined with certain polyoxyalkylene dicarboxylic acid esters can be effective for makeup removing with excellent lathering and be neatly washed out, as well as for washing hairs with refreshing after-shampoo feelings, leading to complete the invention.

### Summary of the Invention

The present invention aims to provide the cleansing agents which are highly effective for makeup removing with excellent lathering and can be neatly washed out, as well as for washing hairs with refreshing after-shampoo feelings.

Namely the present invention provides the cleansing agents comprising surfactants and polyoxyalkylene dicarboxylic acid esters represented by the following general formula (1); 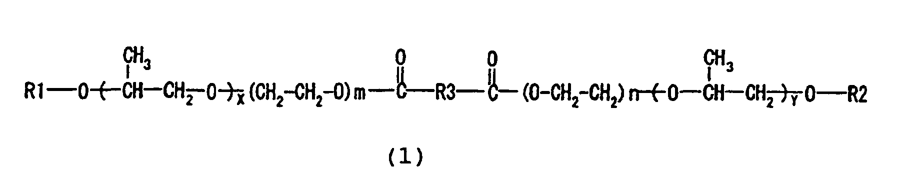 wherein R1 and R2 are hydrogen, or alkyl or branched alkyl groups of from 1 to 4 carbon atoms; m, n, X and Y are integers from 0 to 5 and are not all concurrently 0; and R3 is a branched or straight alkylene group of from 0 to 10 carbon atoms.

Besides, the present invention provides the said cleansing agents, wherein the polyoxyalkylene dicarboxylic acid ester is diethoxyethyl succinate.

Additionally, the present invention provides the cleansing agents, wherein the cleansing agent is a makeup remover, or is a shampoo for washing hairs.

### Detailed Description of the Invention

The present invention is described in details hereinafter.

Polyoxyethylene dicarboxylic acid ester of the above chemical formula (1) used herein is a known compound, but a novel combination component as a cleansing agent ingredient.

In the above general formula (1), R1 and R2 represent alkyl or branched alkyl groups of from 1 to 4 carbon atoms, but when the number of carbon atoms are not less than 5, the cleansing agent has poor lathering due to lack of its hydrophilicity. The symbols, m, n, X and Y represent integers from 0 to 5, preferably from 1 to 2. When the sum of m, n, X and Y represent 0, the cleansing agent has poor lathering due to lack of its hydrophilicity, and when the sum is over 15, the cleansing agent has poor effect on makeup removing due to poor solubility of makeup cosmetics. Besides, R3 represents a branched or straight alkylene group of 0 to 10 carbon atoms, and when having 11 or more carbon atoms, the cleansing agent has poor lathering due to lack of its hydrophilicity.

When polyoxyalkylene dicarboxylic acid ester of the above general formula (1) is diethoxyethyl succinate, the cleansing agents which are highly effective for makeup removing with excellent lathering are especially obtained.

The combined amount of polyoxyalkylene dicarboxylic acid ester is not limited in any way thereto, but is preferably from 0.1 to 10% by weight, more preferably from 1 to 10% by weight based upon the total weight of the cleansing agent. When it is less than 0.1% by weight, the effects of the invention are low, and when the amount is more than 10% by weight, the effects of the invention can not be exerted due to its separation from water.

The surfactants used herein are not limited if the surfactants are generally admitted in cleansing agents.

Examples of fatty acid soaps are potassium laurate, sodium laurate, triethanol ammonium laurate, potassium myristate, sodium myristate, and triethanol ammonium myristate.

Examples of anion surfactants are sodium lauryl sulfate, polyoxyethylene alkyl ether sodium sulfate, sodium alkyl β-alanine, sodium sulfosuccinate, acylmethyl taurine, sodium alkylethane sulfonate, and polyoxyethylene alkyl ether sodium carboxylate.

Examples of cation surfactants are stearyl trimethyl ammonium chloride,benzalkonium chloride,and lauryl amine oxide.

Examples of non ionic surfactants are sorbitan monolaurate, sorbitan monopalmitate, sorbitan sesquioleate, polyoxyethylene sorbitan monolaurate, polyethylene glycol monooleate, polyoxyethylene alkyl ether, polyglycol diester, lauroyl diethanol amide, fatty acid isopropanol amide, maltitol hydroxy fatty acid alkyl ether, alkylated polysaccharide, alkyl glucoside, and sucrose fatty acid ester.

Examples of ampholytic surfactants are amide propyl betaine coconut fatty acid, amide propyl betaine laurate, and amide propyl betaine myristate.

The combined amount of surfactants is not limited in any way thereto, and is appropriately determined for each product, but is preferably 10 to 50% based upon the total weight of the cleansing agent.

The cleansing agent of the present invention means the things having the purpose to cleanse skin of which exodermis is applied for cosmetics, medication and quasi-drugs. Especially, it is preferably used as a makeup remover such as cleansing creams which aims to remove makeup cosmetics. Its formulations can take various forms such as aqueous solutions, emulsions or gels.

The cleansing agent of the present invention can be combined with ingredients generally used for cleansing agents of cosmetics and medications in addition to the above essential components and is produced by standard methods.

Examples of ingredients include as below and the above essential components and one or more of the following components are combined to be manufactured.

Examples of oils are avocado oil, macadamia nut oil, corn oil, olive oil, rape oil, evening primrose oil, castor oil, sunflower seed oil, tea seed oil, rice bran oil, jojoba oil, cacao oil, coconut oil, squalene, beef tallow, Japanese wax, bee wax, candelilla wax, Carnauba wax, whale wax, lanolin, liquid paraffin, polyoxyethylene (8 mole) oleyl alcohol ether, and glyceryl monooleate.

Examples of higher alcohols are caprylic alcohol, lauryl alcohol, myristate alcohol, cetyl alcohol, cholesterol,and phytosterol.

Examples of higher fatty acids are capric acid, lauric acid, myristic acid, palmitic acid, stearic acid, behenylic acid, lanolin fatty acid, linoleic acid,and linolenic acid.

Examples of humectant are polyethylene glycol, glycerin, sorbitol, xylitol, maltitol, mucopolysaccharide, hyaluronic acid, chondroitin sulfate,and chitosan.

Examples of thickening agents are methyl cellulose, ethyl cellulose, gum acacia, and polyvinyl alcohol.

Examples of organic solvents are ethanol, 1,3-butylene glycol.

Examples of anti-oxidants are butyl hydroxy toluene, tocopherol, and phytic acid.

Examples of anti-bacterial preservatives are benzoic acid, salicylic acid, sorbic acid, p-hydroxybenzoate ester (e.g., ethylparaben, butylparaben), and hexachlorophene.

Examples of amino acids and their chlorides are glycin, alanine, valine, leucine, serine, threonine, phenylalanine, tyrosine, aspartic acid, asparagine, glutamine, taurine, arginine, and histidine.

Examples of organic acids are acyl sarcosinic acid (e.g., lauroyl sodium sarcosine), glutathione, citric acid, malic acid, tartaric acid, and lactic acid.

Examples of vitamin Bs are vitamin B6 tripalmitate, vitamin B6 dioctanoate, vitamin B2 and its derivatives, vitamin B12, vitamin B15 and its derivatives. Vitamin Cs are ascorbic acid, ascorbate phosphate ester (salt), and ascorbate dipalmitate.

Examples of vitamin Es are α-tocopherol, β-tocopherol, γ-tocopherol, vitamin E acetate, and vitamin E nicotinate.

Examples of vitamins are vitamin A and its derivatives, vitamin Ds and vitamin H are pantothenic acid and pantethine.

Examples of various agents indicate nicotinic acid amide, nicotinic acid benzyl, γ-orizanol, allantoin, glycyrrhizinic acid (salts), glycyrrhetinic acid and its derivatives, hinokitiol, mucidin, bisabolol, eucalyptol, thymol, inositol, saponins (psycosaponin, carrot saponin, sponge saponin, sapindaceous saponin), pantothenyl ethyl ether, ethynyl estradiol, tranexamic acid, cepharanthin, placenta extract.

Examples of natural extracts extracted with organic solvents, alcohols, polyhydric alcohols, water and aqueous alcohols of such as sorrel, sophora, nuphar, orange, sage, thyme, milfoil, mallow, cnidium rhizone, swertia herb, Japanese angelica root,bitter orange peel, birch,horsetail, sponge gourd, horse chestnut tree, saxifrage, arnica, lily, mugwort, peony root, aloe, gardenia, and Spanish mackerel.

Perfume, scrub agents and purified water and the like can be further combined.

The cleansing agents of the present invention are highly effective for make up removing with excellent lathering and have refreshing after-shampoo feelings.

### EXAMPLES

The examples suitable for the present invention are then described. The present invention is not limited to these examples. The combined amounts are expressed as % by weight unless otherwise indicated.

### Examples 1 to 3, Comparative example 1

The cleansing gels in the Examples and Comparative examples shown in "Table 2" and "Table 3" were produced and subjected to detergency tests of lipstick and foaming power tests. As polyoxyalkylene dicarboxylic acid ester, diethoxyethyl succinate, diethylcarbitol sebacate and ethylethoxyethyl adipate were used.

### "Detergency tests of lipstick"

The lipstick of which formulation was shown in the following "Table 1" was uniformly applied on artificial leather sheets and dried for 10 min.

**[Table 1]**

| A formulation of a lipstick | |
|---|---|
| Combined ingredient | % by Weight |
| 2-Octyl dodecanol | 6.0 |
| Titanium oxide | 1.0 |
| Barium sulfate | 1.0 |
| Mica | 2.0 |
| Vitamin E acetate | 0.1 |
| Ion exchanged water | 0.16 |
| Red color #201 lake pigment | 5.0 |
| Liquid paraffin | 5.0 |
| Solid paraffin | 30.0 |
| Triglyceride caprate/caprylate | 8.0 |
| Propylene glycol dioctanoate | 6.0 |
| 2-Ethylhexyl 12-hydroxystearate | 18.0 |
| 2-Octyldodecyl ricinoleate | 2.0 |
| Ferric oxide | 1.0 |
| Polybutene | 14.74 |

A cotton piece impregnated with 0.1g of each sample shown in Examples 1 to 3 and Comparative example 1 was attached on a reciprocating arm, and the cotton piece on the arm weighted with 50g of load was reciprocated five times on an artificial leather sheet applied with lipstick. Then, the amount of the lipstick on this artificial leather sheet was measured as color differences from colors on unapplied portions using a color-difference meter. The percentage of the removed lipstick amount for the amount of lipstick before being scrubbed with the sample is calculated to give a detergency (%). The formulations of cleansing gels (test samples) in each Example 1 to 3 and Comparative example 1 were shown in "Table 2". The results were also shown in "Table 2".

**[Table 2]**

| Cleansing gels and detergency of lipstick in Examples 1 to 3 and Comparative example 1 | | | | |
|---|---|---|---|---|
| Combined ingredient | Example | | | Comparative Example |
| | 1 | 2 | 3 | 1 |
| Potassium laurate | 5.0 | 5.0 | 5.0 | 5.0 |
| Potassium myristate | 5.0 | 5.0 | 5.0 | 5.0 |
| Amide propylbetaine coconut fatty acid | 5.0 | 5.0 | 5.0 | 5.0 |
| Diethanol amide coconut fatty acid | 3.0 | 3.0 | 3.0 | 3.0 |
| Propylene glycol | 7.0 | 7.0 | 7.0 | 7.0 |
| Glycerin | 5.0 | 5.0 | 5.0 | 5.0 |
| Ion exchanged water | 65.0 | 65.0 | 65.0 | 70.0 |
| Diethoxyethyl succinate | 5.0 | - | - | - |
| Diethylcarbitol sebacate | - | 5.0 | - | - |
| Ethylethoxyethyl adipate | - | - | 5.0 | - |
| Detergency of lipstick | 92 | 87 | 89 | 54 |

As shown in "Table 2", the detergency of lipstick was found to increase by combination of polyoxyalkylene dicarboxylic acid ester of the present invention, especially diethoxyethyl succinate with the formulation of Comparative example 1.

### Foaming test

The cleansing gels were prepared by the formulations in "Table 3". Then, each formulation was dissolved in 70ppm of calcium chloride at 1% , and 40 ml each of this solution was agitated by a mixer for 60 seconds, and the amount of the foam was measured and determined as the foaming power. The formulations were shown in "Table 3" in conjugation with the results.

**[Table 3]**

| Cleansing gels and foaming powers in Examples 1 to 3 and Comparative examples 1 to 3 | | | | | | |
|---|---|---|---|---|---|---|
| Combined ingredient | Example | | | Comparative Example | | |
| | 1 | 2 | 3 | 1 | 2 | 3 |
| Potassium laurate | 5.0 | 5.0 | 5.0 | 5.0 | 5.0 | 5.0 |
| Potassium myristate | 5.0 | 5.0 | 5.0 | 5.0 | 5.0 | 5.0 |
| Propylbetaine coconut fatty acid amide | 5.0 | 5.0 | 5.0 | 5.0 | 5.0 | 5.0 |
| Diethanol palm coconut acid amide | 3.0 | 3.0 | 3.0 | 3.0 | 3.0 | 3.0 |
| Propylene glycol | 7.0 | 7.0 | 7.0 | 7.0 | 7.0 | 7.0 |
| Glycerin | 5.0 | 5.0 | 5.0 | 5.0 | 5.0 | 5.0 |
| Ion exchanged water | 65.0 | 65.0 | 65.0 | 70.0 | 65.0 | 65.0 |
| Diethoxyethyl succinate | 5.0 | - | - | - | - | - |
| Diethylcarbitol sebacate | - | 5.0 | - | - | - | - |
| Ethylethoxyethyl adipate | - | - | 5.0 | - | - | - |
| Liquid paraffin | - | - | - | - | 5.0 | - |
| Cethyl isononanoate | - | - | - | - | - | 5.0 |
| Foaming power (mL) | 2050 | 1900 | 1950 | 2000 | 600 | 800 |

As shown in "Table 3", polyoxyalkylene dicarboxylic acid ester was found not to decrease the foaming power of surfactant as compared with the other oils such as liquid paraffin and cethyl isononanoate. Besides, Diethoxyethyl succinate was found to inhibit lathering least.

As shown in the above, the cleansing agents combined polyoxyalkylene dicarboxylic acid ester with surfactants were found to be highly effective for makeup removing with excellent lathering. Other examples of the present invention are listed as follows.

| Example 4 Cleansing foam | | % by weight |
|---|---|---|
| (1) | Lauric acid | 7.50 |
| (2) | Myristic acid | 2.50 |
| (3) | Stearic acid | 15.00 |
| (4) | Potassium hydroxide | 4.62 |
| (5) | Propylene glycol | 15.00 |
| (6) | Glycerin | 10.00 |
| (7) | Maltitol hydroxy aliphatic (C12, C14) ether | 1.00 |
| (8) | Diethoxyethyl succinate | 1.00 |
| (9) | Perfume | adequately |
| (10) | Purified water | residual |

### (Process of manufacture)

Standard procedures are used. The obtained cleansing foam was highly effective for makeup removing with excellent lathering. Its stability was also good.

| Example 5 Facial cleansing gel | | % by weight |
|---|---|---|
| (1) | Disodium polyoxyethylene sulfosuccinate | 8.0 |
| (2) | Propylbetaine coconut fatty acid amide | 7.0 |
| (3) | Hydroxy lauryl ether sodium acetate | 1.0 |
| (4) | Diethanol amide coconut fatty acid | 2.0 |
| (5) | Citric acid | 1.1 |
| (6) | Diethylcarbitol sebacate | 5.0 |
| (7) | Perfume | adequately |
| (8) | Purified water | residual |

### (Process of manufacture)

Standard procedures are used. The obtained facial cleansing gel was highly effective for makeup removing with excellent lathering. Its stability was also good.

| Example 6 A transparent soap | | % by weight |
|---|---|---|
| (1) | Castor oil | 2.0 |
| (2) | Lauric acid | 6.5 |
| (3) | Myristic acid | 12.0 |
| (4) | Palmitic acid | 3.9 |
| (5) | Stearic acid | 7.6 |
| (6) | Isostearic acid | 2.0 |
| (7) | Sodium hydroxide | 4.0 |
| (8) | Potassium hydroxide | 2.0 |
| (9) | Maltitol | 4.0 |
| (10) | Sorbitol | 10.0 |
| (11) | Glycerin | 11.0 |
| (12) | EDTA-3Na-2H₂O | 0.05 |
| (13) | Ethanol | 10.0 |
| (14) | Diethyl polyoxyethylenyl succinate(3mol) | 2.0 |
| (15) | Perfume | adequately |
| (16) | Purified water | residual |

### (Process of manufacture)

Standard procedures are used. The obtained transparent soap was highly effective for makeup removing with excellent lathering. Its stability was also good.

| Example 7 Liquid facial cleansing agent | | % by weight |
|---|---|---|
| (1) | Potassium laurate | 5.0 |
| (2) | Potassium myristate | 5.0 |
| (3) | Propylbetaine coconut fatty acid amide | 5.0 |
| (4) | Diethanol amide coconut fatty acid | 3.0 |
| (5) | Hydroxy lauryl ether sodium acetate | 4.0 |
| (6) | Maltitol hydroxyalkyl (C12, C14) ether | 2.0 |
| (7) | 1,3-butanediol | 10.0 |
| (8) | Glycerin | 5.0 |
| (9) | Diethoxyethyl succinate | 5.0 |
| (10) | Perfume | adequately |
| (11) | Purified water | residual |

### (Process of manufacture)

Standard procedures are used. The obtained liquid facial cleansing agent was highly effective for makeup removing with excellent lathering. Its stability was also good.

| Example 8 Cleansing gel | | % by weight |
|---|---|---|
| (1) | Methyl alanine sodium coconut oil fatty acid | 50.0 |
| (2) | Propylbetaine laurate amide | 35.0 |
| (3) | Monoethanol amide coconut oil fatty acid | 3.0 |
| (4) | Citric acid | 1.0 |
| (5) | Ethylethoxyethyl adipate | 0.5 |
| (6) | Perfume | adequately |
| (7) | Purified water | residual |

### (Process of manufacture)

Standard procedures are used. The obtained cleansing gel was highly effective for makeup removing with excellent lathering. Its stability was also good.

The cleansing agents for hair in Examples and Comparative examples were also evaluated by the following tests.

### Evaluation (1): smoothness of hairs in washing hairs

The practical use tests were carried out by 10 special panelists to examine hair smoothness in washing hairs. The evaluation criterion are as follows;
++; Eight or more special panelists have agreed with smoothness in washing hairs.
+; Six or more to less than eight special panelists have agreed with smoothness in washing hairs.
±; Three or more to less than six special panelists have agreed with smoothness in washing hairs.
-; Less than three special panelists have agreed with smoothness in washing hairs.

### Evaluation (2) : smoothness of hairs after shampoo and drying

The practical use tests were carried out by 10 special panelists to examine hair smoothness after shampoo and drying, and hair smoothness after natural drying and that before shampoo were sensually evaluated by a comparative method. The evaluation criterion are as follows;
++; Eight or more special panelists have agreed with more smoothness after shampoo.
+; Six or more to less than eight special panelists have agreed with more smoothness after shampoo.
±; Three or more to less than six special panelists have agreed with more smoothness after shampoo.
-; Less than three special panelists have agreed with more smoothness after shampoo.

### Evaluation (3): evaluation of sticky feelings of hairs after shampoo and drying

The practical use tests were carried out by 10 special panelists to examine sticky feelings of hairs after shampoo and drying, and sticky feelings of hairs after natural drying and those before shampoo were sensually evaluated by a comparative method. The evaluation criterion are as follows;
++; All the special panelists did not feel sticky.
+; One or more to less than three special panelists have agreed more sticky hairs after shampoo.
±; Three or more to less than five special panelists have agreed more sticky hairs after shampoo.
-; Five or more of special panelists have agreed more sticky hairs after shampoo.

### Evaluation (4): Foaming power test

Each formulation in Table 4 and 5 each at 3% was dissolved in 70ppm of calcium chloride solution, 400 ml of each mixture was agitated by a mixer for 60 seconds. The amount of foam was measured and determined as the foaming power. The evaluation criterion were as follows;
- ++;: 2400 mL or more
- +;: 2000 mL or more to less than 2400 mL
- ±;: 1500 mL or more to less than 2000 mL
- -;: less than 1500 mL

### Examples 1 to 6, Comparative examples 1 to 4

The cleansing agents for hair comprising combined compositions described in Tables 4 and 5. Examples 1 to 6 and Comparative examples 1 to 4 were manufactured by standard procedures, the evaluation tests for hair were carried out for the above evaluation (1), (2), (3) and (4), and their results were shown in the following Tables.

**Table 4**

| Combined ingredient | Example | | | | | |
|---|---|---|---|---|---|---|
| | 1 | 2 | 3 | 4 | 5 | 6 |
| Sodium cocoyl methyl taurine | 20 | 20 | 20 | 20 | 20 | 20 |
| Diethanol amide coconut fatty acid | 3 | 3 | 3 | 3 | 3 | 3 |
| Sodium benzoate | 0.5 | 0.5 | 0.5 | 0.5 | 0.5 | 0.5 |
| Diethoxyethyl succinate | 0.001 | 0.01 | 0.1 | 1.0 | 5.0 | 15.0 |
| Coloring materials | adq | adq | adq | adq | adq | adq |
| Perfumes | adq | adq | adq | adq | adq | adq |
| Purified water | res | res | res | res | res | res |
| Ev(1) Smoothness in washing hairs | + | + | ++ | ++ | ++ | ++ |
| Ev(2) Smoothness after shampoo | + | + | ++ | ++ | ++ | ++ |
| Ev(3) Sticky feeling after shampoo | ++ | ++ | ++ | ++ | ++ | + |
| Ev(4) Foaming power | ++ | ++ | ++ | ++ | ++ | + |
| adq, adequately; res, residual; Ev, Evaluation | | | | | | |

**Table 5**

| Combined ingredient | Comparative example | | | |
|---|---|---|---|---|
| | 1 | 2 | 3 | 4 |
| Sodium cocoyl methyl taurine | 20 | - | 20 | - |
| Polyoxyethylene (EO average 3 moles) sodium lauryl ether sulfate | - | 20 | - | 20 |
| Diethanol amide coconut fatty acid | 3 | 3 | 3 | 3 |
| Sodium benzoate | 0.5 | 0.5 | 0.5 | 0.5 |
| Diethoxyethyl succinate | - | - | - | - |
| Pentaerythrityl tetraoctanoate | - | - | 5.0 | 5.0 |
| Color materials | adq | adq | adq | adq |
| Perfumes | adq | adq | adq | adq |
| Purified water | res | res | res | res |
| Evaluation (1) smoothness in washing hairs | - | - | ± | + |
| Evaluation (2) smoothness after shampoo | - | - | ± | ± |
| Evaluation (3) sticky feeling after shampoo | + | + | - | - |
| Evaluation (4) Foaming power | ++ | ++ | - | - |
| adq, adequately; res, residual | | | | |

As shown in Tables 4 and 5, the cleansing agents for hair represented in Examples of the present invention are superior in hair feelings, especially smoothness at use and after use and have excellent lather as compared with those in Comparative examples.

## Claims

1. A cleansing agent comprising surfactants and polyoxyalkylene dicarboxylic acid ester represented by the following general formula (1); 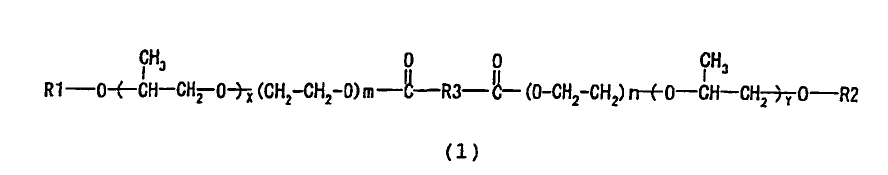 wherein R1 and R2 are hydrogen, or alkyl or branched alkyl groups of 1 to 4 carbon atoms; m, n, X and Y are integers from 0 to 5 and are not all concurrently 0 ; and R3 is a branched or straight alkylene group of 0 to 10 carbon atoms.

2. The cleansing agent according to Claim 1 wherein said polyoxyalkylene dicarboxylic acid ester is diethoxyethyl succinate.

3. The cleansing agent according to Claim 1 or 2 wherein said cleansing agent is a makeup remover.

4. The cleansing agent according to Claim 1 or 2 wherein said cleansing agent is a shampoo for washing hairs.

## Patentansprüche

1. Reinigungsmittel, umfassend Tenside und Polyoxyalkylendicarbonsäureester, dargestellt durch die folgende allgemeine Formel (1) 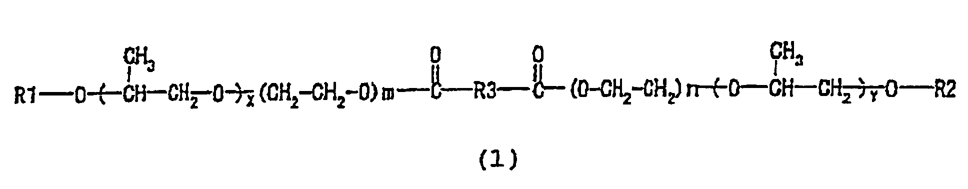 worin R1 und R2 Wasserstoff oder Alkyl- oder verzweigte Alkylgruppen mit 1 bis 4 Kohlenstoffatomen sind; m, n, X und Y ganze Zahlen von 0 bis 5 darstellen und nicht alle gleichzeitig 0 sind; und R3 eine verzweigte oder geradkettige Alkylengruppe mit 0 bis 10 Kohlenstoffatomen ist.

2. Reinigungsmittel nach Anspruch 1, worin der Polyoxyalkylendicarbonsäureester Diethoxyethylsuccinat ist.

3. Reinigungsmittel nach Anspruch 1 oder Anspruch 2, worin das Reinigungsmittel ein Makeup-Entferner ist.

4. Reinigungsmittel nach Anspruch 1 oder Anspruch 2, worin das Reinigungsmittel ein Shampoo zum Waschen der Haare ist.

## Revendications

1. Un agent de nettoyage comprenant des surfactants et un ester d'acide polyoxyalkylène dicarboxylique représenté par la formule générale suivante (1) 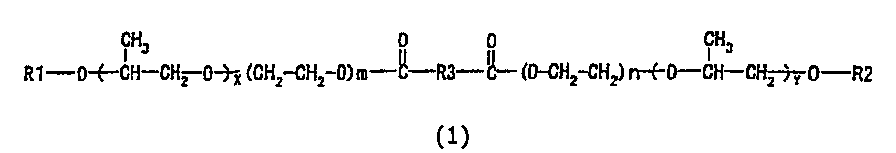 dans laquelle R1 et R2 sont de l'hydrogène ou des groupes alkyl ou alkyl ramifiés, ayant de 1 à 4 atomes de carbone ; m, n, X et Y sont des nombres entiers de 0 à 5 et ne sont pas tous simultanément égaux à 0 ; et R3 est un groupe alkylène ramifié ou linéaire de 0 à 10 atomes de carbone.

2. L'agent de nettoyage selon la revendication 1, dans lequel ledit ester d'acide polyoxyalkylène dicarboxylique est du succinate de diéthoxyéthyle.

3. L'agent de nettoyage selon la revendication 1 ou 2, dans lequel ledit agent de nettoyage est un démaquillant.

4. L'agent de nettoyage selon la revendication 1 ou 2, dans lequel ledit agent de nettoyage est un shampooing pour laver les cheveux.
